# EUROPEAN PATENT APPLICATION

(11) **EP 0 811 695 A2**
(43) Date of publication of application: **10.12.1997**
(21) Application number: 97303816.9
(22) Date of filing: 04.06.1997
(51) Int. Cl.: C12Q 1/68, C12N 15/12

(54) **Mutant S182(PS-1) genes**

(30) Priority: 06.06.1996 US 19222 P
(71) Applicant: WASHINGTON UNIVERSITY, St. Louis, MO 63130 (US); St. Mary's Hospital Medical School, London W2 1PG (GB); University of South Florida, Tampa, Florida 33613 (US)
(72) Inventor: Clark, Robert F., St. Louis, MO 63130 (US); Goate, Alison M., St. Louis, MO 63130 (US); Collinge, John, Imperial College School, London W2 1PG (GB); Hardy, John A., University of South Florida, Tampa, Florida 33613 (US); Hutton, Michael L, University of South Florida, Tampa, Florida 33613 (US)
(74) Representative: Giddings, Peter John, Dr.

(57) **Abstract**

The invention relates to variant PS-1 genes associated with Alzheimer's Disease and methods of detecting the variant genes.

SEQUENCE LISTING

(1) GENERAL INFORMATION

(i) APPLICANT: Clark, Robert
Goate, Alison
Collinge, John
Hardy, John
Hutton, Michael

(ii) TITLE OF THE INVENTION: Mutant S182 (PS-1) Genes

(iii) NUMBER OF SEQUENCES: 16

(iv) CORRESPONDENCE ADDRESS:
(A) ADDRESSEE: SmithKline Beecham Corporation
(B) STREET: 709 Swedeland Road
(C) CITY: King of Prussia
(D) STATE: PA
(E) COUNTRY: USA
(F) ZIP: 19406-0939

(v) COMPUTER READABLE FORM:
(A) MEDIUM TYPE: Diskette
(B) COMPUTER: IBM Compatible
(C) OPERATING SYSTEM: DOS
(D) SOFTWARE: FastSEQ for Windows Version 2.0

(vi) CURRENT APPLICATION DATA:
(A) APPLICATION NUMBER: Unknown
(B) FILING DATE: Herewith
(C) CLASSIFICATION:

(vii) PRIOR APPLICATION DATA:
(A) APPLICATION NUMBER: 60/019,222
(B) FILING DATE: 06-JUN-1996

(viii) ATTORNEY/AGENT INFORMATION:
(A) NAME: Han, William T
(B) REGISTRATION NUMBER: 5219
(C) REFERENCE/DOCKET NUMBER: P50494

(ix) TELECOMMUNICATION INFORMATION:
(A) TELEPHONE: 610-270-5219
(B) TELEFAX: 610-270-5090
(C) TELEX:

(2) INFORMATION FOR SEQ ID NO:1:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: Other

(xi) SEQUENCE DESCRIPTION: SEQ ID NO:1:

TCACAGAAGA TACCGAGACT 20

(2) INFORMATION FOR SEQ ID NO:2:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: Other

(xi) SEQUENCE DESCRIPTION: SEQ ID NO:2:

CCCAACCATA AGAAGAACAG 20

(2) INFORMATION FOR SEQ ID NO:3:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: Other

(xi) SEQUENCE DESCRIPTION: SEQ ID NO:3:

CACCCATTTA CAAGTTTAGC 20

(2) INFORMATION FOR SEQ ID NO:4:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: Other

(xi) SEQUENCE DESCRIPTION: SEQ ID NO:4:

GATGAGACAA GTNCCNTGAA 20

(2) INFORMATION FOR SEQ ID NO:5:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: Other

(xi) SEQUENCE DESCRIPTION: SEQ ID NO:5:

GTGGTAATGT GGTTGGTGAT 20

(2) INFORMATION FOR SEQ ID NO:6:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: Other

(xi) SEQUENCE DESCRIPTION: SEQ ID NO:6:

CCCAACCATA AGAAGAACAG 20

(2) INFORMATION FOR SEQ ID NO:7:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: Other

(xi) SEQUENCE DESCRIPTION: SEQ ID NO:7:

GTGGTAATGT GGTTGGTGAT 20

(2) INFORMATION FOR SEQ ID NO:8:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: Other

(xi) SEQUENCE DESCRIPTION: SEQ ID NO:8:

CCCAACCATA AGAAGAACAG 20

(2) INFORMATION FOR SEQ ID NO:9:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: Other

(xi) SEQUENCE DESCRIPTION: SEQ ID NO:9:

CACCCATTTA CAAGTTTAGC 20

(2) INFORMATION FOR SEQ ID NO:10:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: Other

(xi) SEQUENCE DESCRIPTION: SEQ ID NO:10:

GATGAGACAA GTNCCNTGAA 20

(2) INFORMATION FOR SEQ ID NO:11:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: Other

(xi) SEQUENCE DESCRIPTION: SEQ ID NO:11:

CACCCATTTA CAAGTTTAGC 20

(2) INFORMATION FOR SEQ ID NO:12:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: Other

(xi) SEQUENCE DESCRIPTION: SEQ ID NO:12:

GATGAGACAA GTNCCNTGAA 20

(2) INFORMATION FOR SEQ ID NO:13:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: Other

(xi) SEQUENCE DESCRIPTION: SEQ ID NO:13:

CACCCATTTA CAAGTTTAGC 20

(2) INFORMATION FOR SEQ ID NO:14:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: Other

(xi) SEQUENCE DESCRIPTION: SEQ ID NO:14:

GATGAGACAA GTNCCNTGAA 20

(2) INFORMATION FOR SEQ ID NO:15:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: Other

(xi) SEQUENCE DESCRIPTION: SEQ ID NO:15:

GTGGTAATGT GGTTGGTGAT 20

(2) INFORMATION FOR SEQ ID NO:16:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: Other

(xi) SEQUENCE DESCRIPTION: SEQ ID NO:16:

CCCAACCATA AGAAGAACAG 20

## Description

### RELATED APPLICATION

This application claims the benefit of U.S. Provisional Application No. 60/019,222, filed June 6, 1996.

### BACKGROUND OF THE INVENTION

Alzheimer's disease (AD) is a progressive degenerative disease of the central nervous system characterized clinically by dementia and neuropathologically by the presence of numerous senile plaques and neurofibrillary tangles. AD is typically a disease of the elderly, afflicting up to 6% of those aged 65 years and up to 20% of 80 years olds. This is termed late onset AD. In addition, a small number of pedigrees have been described wherein the disease is inherited as an autosomal dominant with age dependent penetrance. Most commonly, the age of onset of the disease is below 60 years in these families. This is termed early onset AD. Genetic factors have been implicated in both early and late onset AD.

At least three different genetic loci that confer inherited susceptibility to this disease have been identified.

The ε4 (112 Cys→Arg) allele of the apolipoprotein E (*ApoE*) gene located on chromosome 19 is associated with AD in a significant proportion of cases with late onset. Strittmatter et al. *Proc. Nat'l Acad. Sci. USA* 1993 90:1977-1981; Saunders et al. *Neurology* 1993 43:1467-1472. Inheritance of this gene has also been reported to lower the age of onset. Corder et al. *Science* 1993 **261**:921-923. Conversely, inheritance of the apolipoprotein E ε2 allele appears to confer a decreased risk of developing AD. Corder et al. *Nature Genet.* 1994 **7**:180-184. While the biochemical mechanism of these effects is still unclear, there is a significantly increased number of Aβ deposits in the brain in patients having one or two ε4 alleles. Schmechel et al. *Proc. Nat'l Acad. Sci. USA* 1993 **90**:9649-9653; Hyman et al. *Proc. Nat'l Acad. Sci. USA* 1995 **92**:3586-3590.

Mutations in the gene for the β-amyloid precursor protein (βAPP) on chromosome 21 have been found in a small number of families with disease onset before 65 years of age. Goate et al. *Nature* 1991 **349**:704-706; Chartier-Harlin et al. *Nature* 1991 **353**:844-846; Murrell et al. *Science* 1991 **254**:97-99; Karlinsky et al. *Neurology* 1992 **42**:1445-1453. These disease-causing mutations have been modelled in transfected or primary cultured cells and shown to lead to altered proteolytic processing of βAPP in a way that favors production of its amyloidogenic and potentially neurotoxic Aβ fragment. Further, transgenic overexpression of one mutant βAPP has resulted in the first mouse model of AD, in which age-linked cerebral deposition of Aβ is accompanied by neuronal, astrocytic and microglial pathology. Games et al. *Nature* 1995 **373**:523-527.

A third locus (AD3) has been mapped by genetic linkage studies to chromosome 14q24.3 which may account for up to 70% of early-onset autosomal dominant AD. Schellenberg et al. *Science* 1992 **258**:668-670; St. George-Hyslop et al. *Nature Genet.* 1992 **2**:330-334; Van Broeckhoven et al. *Nature Genet.* 1992 **2**:335-339. The AD3 locus is associated with the most aggressive form of this disease (onset 30 to 60 years of age) and it has been suggested that mutations at this locus put into effect a biologically fundamental process leading to AD. Sherrington et al. *Nature* 1995 **375**:754-760.

A novel gene with five missense mutations in seven pedigrees segregating early-onset, autosomal dominant AD on the AD3 locus was recently cloned. Sherrington et al. *Nature* 1995 **375**:754-760. This gene has been called S182 (or PS-1). Analysis of the nucleotide sequences of the S182 transcript revealed heterozygous nucleotide substitutions in the reverse transcriptase-polymerase chain reaction products from affected members of six large pedigrees. Each of the nucleotide substitutions occurred within a putative open reading frame of the S182 transcript and are predicted to change the encoded amino acid at the following positions (numbering from the first putative initiation codon) Met→Leu at codon 142 in pedigrees FAD4 and Tor1.1; His→Arg at codon 163 in pedigree 603; Ala→Glu at codon 246 in pedigree FAD1; Leu→val at codon 286 in pedigree FAD2; and Cys→Try at codon 410 in pedigrees FAD3 and NIH2. Of all the nucleotide substitutions cosegregated with the disease in their respective pedigrees, none were seen in asymptomatic family members aged more than 2 standard deviations beyond the mean onset, and none were present on 284 chromosomes from unrelated neurologically normal subjects drawn from comparable ethnic origins. Sherrington et al. *Nature* 1995 **375**:754-760.

A number of other mutant S182 genes have now been identified. These mutants are useful in the diagnosis of early-onset AD and in evaluating agents which may be useful for the treatment of this disease.

### SUMMARY OF THE INVENTION

An object of the present invention is to provide novel mutant S182 sequences.

Another object of the present invention is to provide a method of diagnosing Alzheimer's disease using these novel mutant S182 genes.

Yet another object of the present invention is to provide a model system for Alzheimer's disease comprising a mutant S182 gene.

### DETAILED DESCRIPTION OF THE INVENTION

Genetic linkage strategies placed a gene causing early onset familial Alzheimer's disease (FAD) on the long arm of chromosome 14 between D14S289 and D14S61. Five mutations within the S182 gene, which maps to this region, were recently reported in several families multiply affected by early onset AD. Sherrington et al. *Nature* 1995 **375**:754-760. This gene has now been localized to a 100 kb region between previously identified markers, D14S77 and D14S668E. The intron-exon structure of this gene has now been determined along with evidence for alternate splicing. In addition, a number of novel mutants in the S182 gene have now been identified in families multiply affected by early onset AD.

Nucleotide substitutions were identified in the S182 transcript which are predicted to change the encoded amino acid at the following positions (numbering from the first putative initiation codon) Met→Val at codon 139 in pedigrees F148 and F206; and Met→Val at codon 146 in pedigrees Haltia. In each case, the mutations have been identified only in afflicted members of each family. These mutations do not coincide with any of those described by Sherrington et al. *Nature* 1995 **375**:754-760.

Fifty families multiply affected by early onset AD were screened for mutations. Of these fifty families only one family, which was believed to be a branch of FAD4, had a published mutation. However, five of these families demonstrated digest changes.

The family described by Haltia et al. Annals of Neurol. 1994 36:362-367 showed a change in the restriction pattern generated by the enzyme RcaI with primers 910/911. Primers 910: 5'-TCACAGAAGATACCGAGACT-3' (SEQ ID NO:1) and 911: 5'-CCCAACCATAAGAAGAACAG-3' (SEQ ID NO:2) are disclosed by Sherrington et al. in *Nature* 1995 **375**:754-760. Direct sequencing of affected members of samples from this family revealed a mutation (A→G) which occurs at the same nucleotide as the FAD4 mutation, but results in a different amino acid substitution, methionine to valine at codon 142 (referred to as codon 146 by Sherrington et al.).

A sample from F148 (Mullan et al. *Nature Genet.* 1992 **2**:340-343) also showed a change in digest pattern with the same enzyme and primers. However, this pattern was different. Direct sequencing revealed a change from A→G at codon 135 resulting in a predicted amino acid change of methionine to leucine. This same change was also observed in F206 (Mullan et al. *Nature Genet.* 1992 **2**:340-343) and segregated with the disease in both families.

The identification of these mutants provides strong evidence that mutations in the S182 gene are the cause of early onset Alzheimer's disease in Chromosome 14-linked pedigrees. These mutants can be used in the diagnosis of AD and also in the development of models of AD.

At present there is no known effective therapy for the various forms of AD. However, there are several other forms of dementia for which treatment is available and which give rise to progressive intellectual deterioration closely resembling the dementia associated with Alzheimer's disease. A diagnostic test for AD would therefore provide a useful tool in the diagnosis and treatment of these other conditions, by way of being able to exclude early onset Alzheimer's disease. It will also be of value when a suitable therapy for AD does become available.

There are several methodologies available from recombinant DNA technology which may be used for detecting and identifying genetic mutations responsible for Alzheimer's disease. These include, but are not limited to, direct probing, ligase chain reaction (LCR) and polymerase chain reaction (PCR) methodology.

Detection of point mutations using direct probing involves the use of oligonucleotide probes which may be prepared synthetically or by nick translation. In a preferred embodiment, the probes are complementary to at least a portion of the mutant S182 genes, said portion containing the mutation, identified herein. The DNA probes may be suitably labelled using, for example, a radiolabel, enzyme label, fluorescent label, or biotin-avidin label, for subsequent visualization in for example a Southern blot hybridization procedure. The labelled probe is reacted with a sample of DNA from a patients suspected of having AD bound to nitrocellulose or Nylon 66 substrate. The areas that carry DNA sequences complementary to the labeled DNA probe become labelled themselves as a consequence of the reannealing reaction. The areas of the filter that exhibit such labeling may then be visualized, for example, by autoradiography.

Alternative probe techniques, such as ligase chain reaction (LCR) involve the use of a mismatch probe, i.e., probes which have full complementary with the target except at the point of the mutation. The target sequence is then allowed to hybridize both with the oligonucleotides having full complementarity, i.e., oligonucleotides complementary to the S182 mutants of the present invention, and oligonucleotides containing a mismatch under conditions which will distinguish between the two. By manipulating the reaction conditions it is possible to obtain hybridization only where there is full complementarity. If a mismatch is present then there is significantly reduced hybridization.

The polymerase chain reaction (PCR) is a technique that amplifies specific DNA sequences. Repeated cycles of denaturation, primer annealing and extension carried out with a heat stable enzyme Taq polymerase leads to exponential increases in the concentration of desired DNA sequences.

Given the knowledge of nucleotide sequences encoding the S182 gene, it is possible to prepare synthetic oligonucleotides complementary to the sequences which flank the DNA of interest. Each oligonucleotide is complementary to one of the two strands. The DNA is then denatured at high temperatures (e.g. 95°C) and then reannealed in the presence of a large molar excess of oligonucleotides. The oligonucleotides, oriented with their 3' ends pointing towards each other, hybridize to opposite strands of the target sequence and prime enzymatic extension along the nucleic acid template in the presence of the four deoxyribonucleotide triphosphates. The end product is then denatured again for another cycle. After this three-step cycle has been repeated several times, amplification of a DNA segment by more than one million fold can be achieved. The resulting DNA may then be directly sequenced in order to locate any genetic alterations. Alternatively, the identified S182 mutants of the present invention make it possible to prepare oligonucleotides that will only bind to altered DNA, so that PCR will only result in the multiplication of the DNA if the mutation is present. Following PCR, allele-specific oligonucleotide hybridization may be used to detect the AD point mutation.

Alternatively, an adaptation of PCR called amplification of specific alleles (PASA) can be employed; this method uses differential amplification for rapid and reliable distinction between alleles that differ at a single base pair. Newton et al. *Nucleic Acid Res.* 1989 **17**:2503; Nichols et al. *Genomics* 1989 **5**:535; Okayama et al. *J. Lab. Clin. Med.* 1989 **1214:**105; Sarkar et al. *Anal. Biochem.* 1990 **186**:64; Sommer et al. *Mayo Clin. Proc.* 1989 **64**:1361; Wu *Proc. Nat'l Acad. Sci. USA* 1989 **86**:2757; and Dutton et al. *Biotechniques* 1991 **11**:700. PASA involves amplification with two oligonucleotide primers such that one is allele specific. The desired allele is efficiently amplified, while the other allele(s) is poorly amplified because it mismatches with a base at or near the 3' end of the allele specific primer. Thus, PASA or the related method PAMSA can be used to specifically amplify one or more variant S182 alleles. Where such amplification is performed on genetic material obtained from a patient, it can serve as a method of detecting the presence of one or more variant S182 alleles in a patient.

Also important, is the development of experimental models of Alzheimer's disease. Such models can be used to screen for agents that alter the degenerative course of AD. Having identified specific mutations in the S182 gene as a cause of early onset familial Alzheimer's disease, it is possible using genetic manipulation, to develop transgenic model systems and/or whole cell systems containing a mutated S182 gene or a portion thereof. The model systems can be used for screening drugs and evaluating the efficacy of drugs in treating Alzheimer's disease. In addition, these model systems provide a tool for defining the underlying biochemistry of S182 and its relationship to AD thereby providing a basis for rational drug design.

One type of cell system which can be used in the present invention can be naturally derived. For this, blood samples from an affected individual are obtained and permanently transformed into a lymphoblastoid cell line using, for example, Epstein-Barr virus. Once established, such cell lines can be grown continuously in suspension cultures and can be used in a variety of *in vitro* experiments to study S182 expression and processing.

Since the FAD mutation is dominant, an alternative method for constructing a cell line is to genetically engineer a S182 mutated gene, or portion thereof, as described herein, into an established cell line of choice. Such methods are well known in the art as exemplified by Sisodia *Science* 1990 248:492 and Oltersdork et al. J. *Biol. Chem.* 1990 **265**:4492 wherein an amyloid precursor peptide gene was transfected into mammalian cells.

Baculovirus expression systems have also been found to be useful for high level expression of heterologous genes in eukaryotic cells.

The mutated gene can also be excised for use in the creation of transgenic animals containing the mutated gene. For example, a mutant S182 gene of the present invention can be cloned and placed in a cloning vector. Examples of cloning vectors which can be used include, but are not limited to, λCharon35, cosmid, or yeast artificial chromosome. The mutant S182 gene can then be transferred to a host nonhuman animal such as a mouse. As a result of the transfer, the resultant transgenic nonhuman animal will preferably express one or more of the variant S182 polypeptides.

Alternatively, minigenes encoding variant S182 polypeptides can be designed. Such minigenes may contain a cDNA sequence encoding a variant S182 polypeptide, preferably full-length, a combination of S182 exons, or a combination thereof, linked to a downstream polyadenylation signal sequence and an upstream promoter (and preferably enhancer). Such a minigene construct will, when introduced into an appropriate transgenic host, such as a mouse or rat, express a variant S182 polypeptide.

One approach to creating transgenic animals is to target a mutation to the desired gene by homologous recombination in an embryonic stem (ES) cell *in vitro* followed by microinjection of the modified ES cell line into a host blastocyst and subsequent incubation in a foster mother. Frohman and Martin *Cell* 1989 **56**:145. Alternatively, the technique of microinjection of the mutated gene, or portion thereof, into a one-cell embryo followed by incubation in a foster mother can be used. Additional methods for producing transgenic animals are well known in the art.

Transgenic animals are used in the assessment of new therapeutic compositions and in carcinogenicity testing as exemplified by U.S. Patent 5,223,610. These animals are also used in the development of predictive animal models for human disease states as exemplified in U.S. Patent 5,221,778. Transgenic animals have now been developed for assessing Alzheimer's disease (U.S. Patent 7,769,626), multi-drug resistance to anticancer agents (U.S. Patent 7,260,827), and carcinogenic substances (U.S. Patent 4,736,866). Therefore, the mutated genes of the present invention which are believed to cause early onset Alzheimer's disease in Chromosome 14-linked pedigrees provide a useful means for developing transgenic animals to assess this disease.

Site directed mutagenesis and/or gene conversion can also be used to a mutate a non human S182 gene allele, either endogenously or via transfection, such that the mutated gene encodes a S182 polypeptide with an altered amino acid as described in the present invention.

### SPECIFIC EXPERIMENTS

In the present application, the nucleotide indicated by the letter N can be selected from the group consisting of A, C, G and T.

### Example 1: Identification of R269G mutation (G -> C)

A novel mutation was identified in exon 8 of S182 at codon 269 (arginine-glycine) arising from a G to C transition (GGT -> CGT). R269G was found by PCR. More typically 100 µl reaction mix contained water, 10X buffer, 10 mM dNTP's, taq polymerase and 20 µM primer 5'-CACCCATTTACAAGTTTAGC-3' (SEQ ID NO: 3) and 5'-GATGAGACAAGTNCCNTGAA-3' (SEQ ID NO: 4). The PCR conditions were 94°C for 5 minutes, (94°C for 30 seconds, 50°C for 30 seconds, 72°C for 45 seconds) x 35, 72°C for 10 minutes. The PCR products were electrophoresed on a 3% agarose gel and visualized by ethidium bromide. The biotinylated primer allows single stranded DNA to be derived using streptavidin-coated magnetic beads and sequenced on an ALF sequencer (Pharmacia)using Autoread kits (Pharmacia).

Similarly, the PCR method of the above Example can be followed to detect additional mutations in the PS-1 gene which are listed in the following Table along with primers to be used and renaturation conditions.

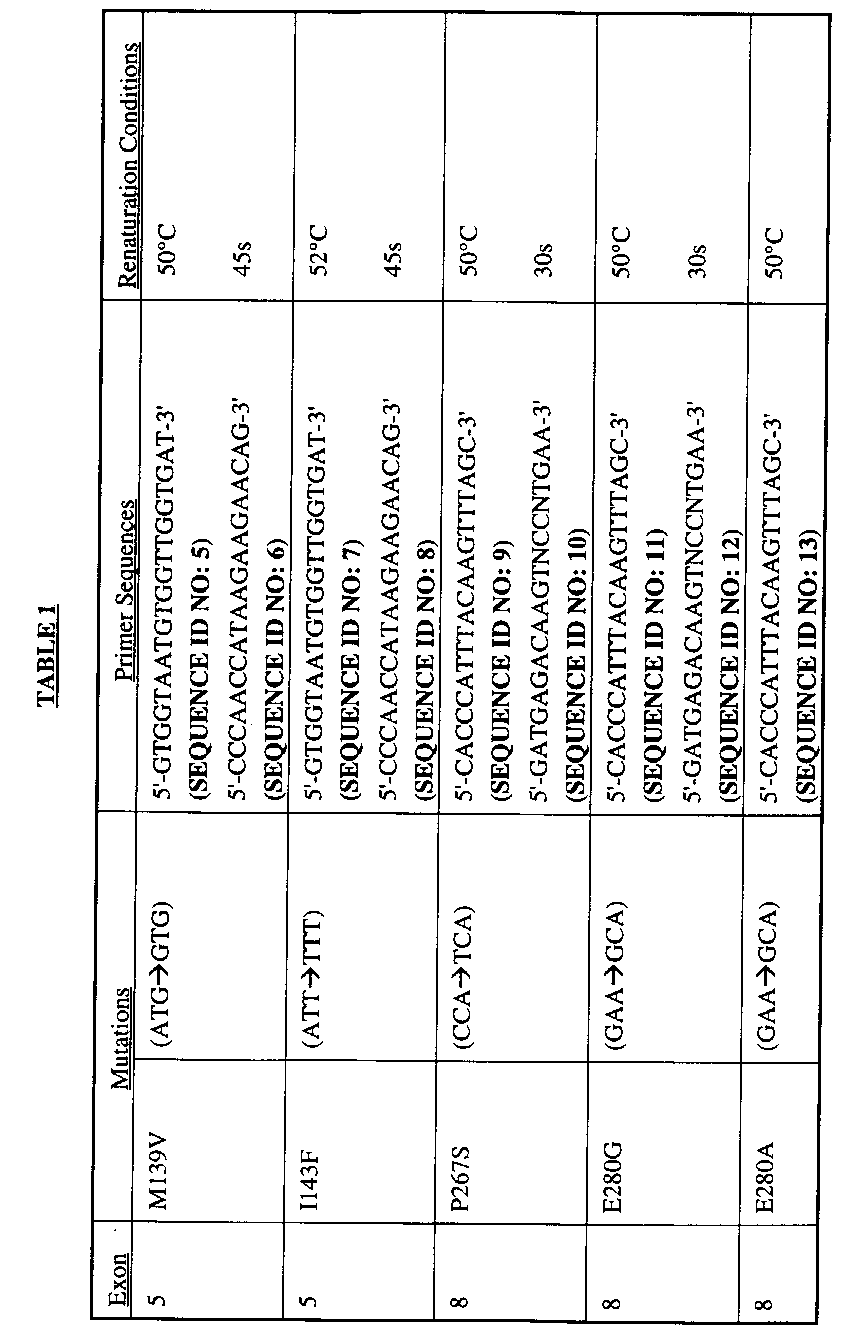

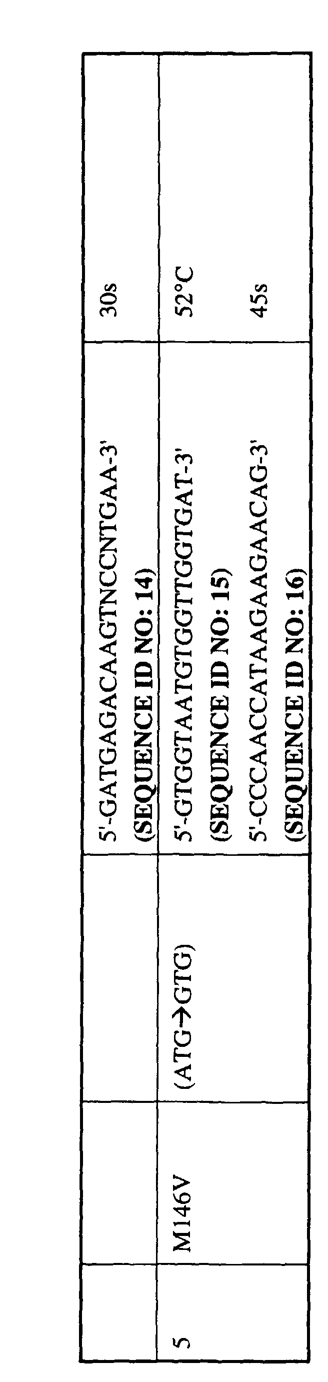

## Claims

1. A variant PS-1 gene comprising the members from:
(a) mutation at codon 139;
(b) mutation at codon 143;
(c) mutation at codon 267;
(d) mutation at codon 280;
(e) mutation at codon 280;
(f) mutation at codon 146; and
(g) mutation at codon 269.

2. A method of detecting a variant PS-1 gene by amplifying a portion of the PS-1 gene containing a point mutation by using a PCR method and sequencing the amplified sequence.

3. A method as claimed in Claim 2 in which a pair of PCR primers used is comprised of oligonucleotides of SEQUENCE ID NOS. 3 and 4.

4. A method as claimed in Claim 2 in which a pair of PCR primers used is comprised of oligonucleotide SEQUENCE ID NOS. 5 and 6.

5. A method as claimed in Claim 2 in which a pair of PCR primers used is comprised of oligonucleotides of SEQUENCE ID NOS. 7 and 8.

6. A method as claimed in Claim 2 in which a pair of PCR primers used is comprised of oligonucleotide SEQUENCE ID NOS. 9 and 10.

7. A method as claimed in Claim 2 in which a pair of PCR primers used is comprised of oligonucleotides of SEQUENCE ID NOS. 11 and 12.

8. A method as claimed in Claim 2 in which a pair of PCR primers used is comprised of oligonucleotide SEQUENCE ID NOS. 13 and 14.

9. A method as claimed in Claim 2 in which a pair of PCR primers used is comprised of oligonucleotides of SEQUENCE ID NOS. 15 and 16.

10. Oligonucleotide of SEQUENCE ID NO. 3.

11. Oligonucleotide of SEQUENCE ID NO. 4.

12. Oligonucleotide of SEQUENCE ID NO. 5.

13. Oligonucleotide of SEQUENCE ID NO. 6.

14. Oligonucleotide of SEQUENCE ID NO. 7.

15. Oligonucleotide of SEQUENCE ID NO. 8.

16. Oligonucleotide of SEQUENCE ID NO. 9.

17. Oligonucleotide of SEQUENCE ID NO. 10.

18. Oligonucleotide of SEQUENCE ID NO. 11.

19. Oligonucleotide of SEQUENCE ID NO. 12.

20. Oligonucleotide of SEQUENCE ID NO. 13.

21. Oligonucleotide of SEQUENCE ID NO. 14.

22. Oligonucleotide of SEQUENCE ID NO. 15.

23. Oligonucleotide of SEQUENCE ID NO. 16.
